# EUROPEAN PATENT APPLICATION

(11) **EP 1 260 211 A1**
(43) Date of publication of application: **27.11.2002**
(21) Application number: 01904534.3
(22) Date of filing: 19.02.2001
(51) Int. Cl.: A61K 7/00, A61K 7/48

(54) **SKIN PREPARATIONS FOR EXTERNAL USE**

(30) Priority: 21.02.2000 JP 2000043366; 09.02.2001 JP 2001033212
(71) Applicant: PENTAPHARM Ltd., 4002 Basel (CH); Ezaki Glico Company Limited, Osaka-shi, Osaka 555-0021 (JP)
(72) Inventor: NAKAYAMA, Hiroki, Shinjuku-ku, Tokyo 169-0073 (JP); OKADA, Shigetaka, Ikoma-shi, Nara 630-0213 (JP); KOMETANI, Takashi, Itami-shi, Hyogo 664-0846 (JP); NISHIMURA, Takahisa, Nara-shi, Nara 631-0833 (JP)
(74) Representative: Braun, André
(86) International application number: JP0101155
(87) International publication number: WO01060324

(57) **Abstract**

As dermatological topical preparations formulated therein melanin production inhibitors which, unlike the conventional melanin production inhibitors, are stable with respect to heat and oxidation and which do no cause decomposition or pigmentation over time when added to topical agents, hydroquinone-α-D-glucopyranoside, which shows superior inhibitory activities on human tyrosinase, is added as a melanin production inhibitor.

## Description

### Technical field

This invention is related to dermatological topical preparations containing hydroquinone-α-D-glucopyranoside, a compound obtained as a result of the α-binding of glucose to one of the OH groups of hydroquinone.

Dermatological topical preparations include basic skin care products such as cream, lotion, essence, facial mask, cold cream and makeup base, cosmetics for make-up such as foundation, blusher, eye shadow, eyeliner and nail lacquer, hair care products such as shampoo and hair tonic, and body care products such as facial scrub, body soap, bath agent, soap and fragrance.

### Technical background

Various melanin production inhibitors have been used to whiten the skin (prevention of skin blackening) and to prevent or improve liver spots, freckles and other skin troubles caused by excessive exposure to UV rays. For example, representative examples thereof may include hydroquinone, arbutin which is obtained as a result of the β-binding of glucose to the phenolic OH group of hydroquinone (β-arbutin), vitamin C and its derivatives, and kojic acid.

However, vitamin C, hydroquinone and kojic acid are very unstable with respect to heat and oxidation, in particular in water, and presents problems such as decomposition and coloration over time when used in dermatological topical preparations.

Derivatives of vitamin C, such as L-ascorbyl magnesium phosphate, and β-arbutin are more stable with respect to heat and oxidation, but their efficacy is not necessarily satisfactory.

### Object of the invention

An object of the present invention is to present dermatological topical preparations of melanin production inhibitors which completely or partly solve these problems with the conventional melanin production inhibitors.

### Summary of the invention

In an effort to solve these problems, the present inventors looked for substances which are stable in topical preparations, safe and much more effective than conventional substances and obtained the following findings:
(1) First, as a compound which meets the above-mentioned object, there exists hydroquinone-α-D-glucopyranoside (hereafter referred to as α-arbutin).
(2) α-Arbutin specifically and strongly inhibits human tyrosinase.
(3) α-Arbutin is more stable and therefore safer than β-arbutin, a commercially available whitening agent.

The dermatological topical preparations of the present invention are based on the above-mentioned findings and are characterized by the fact that they contain α-arbutin, which shows excellent inhibitory activity against human tyrosinase.

Usually, hydroquinone-β-glucopyranoside is referred to as arbutin, andhydroquinone-α-D-glucopyranoside, shown in the following chemical formula, has a structure completely different from that of this arbutin. In the present invention, however, hydroquinone-α-D-glucopyranoside will be referred to as α-arbutin in order to clearly distinguish it from the above-mentioned arbutin, and the above-mentioned arbutin will be referred to as β-arbutin.

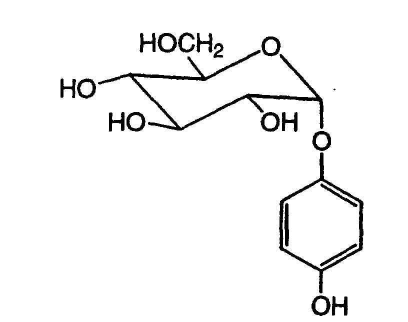

The α-arbutin is obtained, for example, through the glycosidization of hydroquinone using an enzyme obtained from bacteria. This enzyme is α-amylase, and may be Amylase X-23 disclosed in Japanese Patent No. 2662667.

The enzyme tyrosinase plays the most important role in the formation of melanin, which causes pigmentation. Most of the currently available whitening agents and other topical agents designed to prevent freckles and improve liver spots contain tyrosinase inhibitors.

The strength of tyrosinase inhibiting activity is closely related to the extent to which the above-mentioned objects are achieved. The greater the tyrosinase inhibiting activity, the greater whitening effects and the greater effect to prevent freckles and improve liver spots can be expected.

Mushroom-derived tyrosinase has been commonly used to assess the tyrosinase inhibiting activity.

According to the data described in Biochem. Biotech. Biochem., 59, 143 (1995), however, α-arbutin does not show tyrosinase inhibiting activity in this conventional evaluation system.

Furthermore, Tokkaihei (Japanese Provisional Patent Publication) No. 5-932 concludes that α-arbutin promotes, rather than inhibits, mushroom-derived tyrosinase activity and that it is effective as a suntanning agent.

According to Tokkaihei No. 6-153976, there is disclosed that α-arbutin is effective in inhibiting mushroom-derived tyrosinase.

On the other hand, like a key and a keyhole, enzymes are very specific and interact only with specific substrates. The same is true for enzyme inhibitors. For example, Agric. Biol. Chem., 44, 1683 (1980) describes α-amylase inhibitors which affect α-amylase deriving from animals, but not α-amylase deriving from plants or microorganisms. As is shown by these α-amylase inhibitors, a substance which inhibits a specific enzyme may not inhibit other enzymes from different origins.

Thus, controversial results have been reported in the assessment of α-arbutin's inhibitory effects on tyrosinase using mushroom-derived tyrosinase, and it is obvious that the assessment system using mushroom-derived tyrosinase cannot be used to assess the effects on tyrosinase from other animals and plants.

The present inventors paid attention to the specificity of each enzyme from the viewpoint of enzymatic chemistry and thought, as mentioned above, that the conventional assessment system using mushroom-derived tyrosinase is not appropriate for assessing whitening agents and other preparations intended for use in humans. Because whitening agents and other preparations are used in humans, the present inventors concluded that the inhibitory effects of α-arbutin of the present invention should be assessed using tyrosinase from humans and conducted studies using human-derived tyrosinase.

Specifically, tyrosinase obtained from cultured human skin cells was purified and used to assess the inhibitory activity of α-arbutin on human tyrosinase. Results obtained showed that α-arbutin exerts specific, very strong inhibitory effects on tyrosinase from humans.

Based on this finding, the present inventors conducted clinical trials in volunteers in order to determine if dermatological topical preparations containing α-arbutin according to the present invention show whitening effects (prevention of skin blackening) and improve liver spots. Dermatological topical preparations containing α-arbutin of the present invention showed much greater effects than conventional agents used to inhibit melanin formation.

Hydroquinone, which is produced as a result of the decomposition of β-arbutin, a conventional melanin-formation inhibitor, has been reported to be about 400 times more cytotoxic than β-arbutin.

α-Arbutin has been confirmed to be more stable than β-arbutin and is much less unlikely to produce hydroquinone. It is therefore safer than β-arbutin.

On the other hand, as a method for producing an α-arbutin, the present inventors proposed a method in which α-amylase (for example, amylase X-23) deriving from Bacillus subtilis (for example, X-23 (Seikoken (National Institute of Bioscience and Human-Technology) strain deposited P-13560) is used as an enzyme and glucans with α-1,4 binding such as malt oligosaccharide, amylose, amylopectin, and various types of starch are used as sugar donors (see Japanese Patent Publication No. 2662667).

α-arbutin obtained using this published method inhibits the activity of tyrosinase from humans much more strongly than the conventional β-arbutin mentioned above. As shown in the examples later, α-arbutin is also safer than β-arbutin when used in humans and is very stable with respect to heat and oxidation in both air and water. When used in dermatological topical preparations, it is not decomposed or dose not cause pigmentation over time.

α-Arbutin, which exerts potent inhibitory activity against tyrosinase derived from humans, which is safe in humans and stable with respect to heat and oxidation in air and water, and which is not altered over time in dermatological topical preparations as mentioned above, can be industrially mass-produced under the conditions outlined below, for example, using the above-mentioned α-amylase and sugar donors (soluble starch in order to efficiently obtain α-arbutin on an industrial scale).

Dissolve hydroquinone so that it is 1 to 20% by weight, preferably 1 to 15% by weight, and soluble starch so that it is 1 to 20% by weight, preferably 5 to 10% by weight in a solvent (aqueous solution containing phosphoric acid, acetic acid, etc.) of pH 4 to 9, preferably pH 5 to 8, heat the solution obtained up to room temperature to 90°C, preferably 30 to 70°C, add the above-mentioned α-amylase in an amount of 1 to 100 units, preferably 5 to 80 units, and allow the solution to react for 1 to 48 hours, preferably 5 to 24 hours, at the above-mentioned temperature.

The yield can be increased by, for example, adding 10 to 50% of the above-mentioned amount of soluble starch at first and adding the remainder gradually in the reaction process (for example, designated time points every 1 to 5 hours), or by adding 50% of the above-mentioned amount of soluble starch at first and adding the remainder in the middle of the reaction process.

The present invention is related to dermatological topical preparations containing α-arbutin, which is obtained as mentioned above and which presents the above-mentioned characteristics, and these preparations include ointment, cream, lotion, etc. but are not limited to these dosage forms.

The dermatological topical preparations of the present invention produce good whitening effects (prevention of the blackening of the skin) and effectively prevent freckles and improve liver spots when the content of α-arbutin is 0.05 to 10% by weight, preferably 0.05 to 2% by weight of the total amount of the dermatological topical preparations, but these contents of α-arbutin by no means restrictively define the present invention.

### Brief explanations of the drawings

Fig. 1 is a graph showing comparative test results obtained regarding the stability of the α-arbutin of the present invention and β-arbutin on the mouse skin.
Fig. 2 is a graph showing another comparative test results obtained regarding the stability of the α-arbutin of the present invention and β-arbutin on the mouse skin.

### Examples

### [Preparation of α-arbutin]

Hydroquinone (10% by weight) and maltpentose (20% by weight) were dissolved in 20mM acetic acid in water (pH5.5), and 10 mL (in the following, milliliter is referred to as "mL" and liter is referred to as "L") of an enzyme solution containing 60 units of α-amylase (amylase X-23) was added to 10 mL of the solution obtained.

The mixture obtained was maintained at 40°C for 16 hours, and 3-times volume of ethyl acetate were added. The mixture was violently shaken and allowed to stand at room temperature for 30 minutes before collecting an aqueous fraction. These procedures were repeated 3 times, and the aqueous fraction was dried under reduced pressure after completely removing hydroquinone which did not react.

The dried material obtained was dissolved in purified water, and the glycoside was allowed to be adsorbed to an active carbon column equilibrated with purified water.

The column was first treated with 20% methanol to elute sugar which did not react and was adsorbed to the column. The column was then treated with 100% methanol to elute glycoside.

The 100% methanol fraction was dried under reduced pressure, dissolved again in purified water and then freeze-dried.

About 200 mg of the glycoside of hydroquinone was obtained through these procedures.

In this case, the sugar transfer rate was about 27%.

The sugar transfer rate increased to about 40% when maltpentose was added to the above-mentioned aqueous solution of acetic acid at 5% by weight, 6 hours after starting reaction.

### [Preparation example of dermatological topical preparation]

### Formulation example 1

To prepare a preparation of the composition shown in Table 1, the α-arbutin prepared in the above-mentioned example (hereafter the term "α-arbutin" shall always mean this α-arbutin), methyl parahydroxybenzoate, and butyl parahydroxybenzoate were added to a mixture of purified water and propylene glycol, and the mixture obtained was heated up to 80°C to dissolve.

To the solution obtained, a mixture of the other ingredients shown in Table 1, homogeneously dissolved by heating up to 80°C, was gradually added, and the mixture obtained was emulsified at a high speed using a homo-mixer.

After emulsification, the mixture was gradually cooled down to 30°C with stirring to prepare an ointment.

**Table 1**

| | % by weight |
|---|---|
| α-Arbutin | 2.0 |
| Vaseline | 4.0 |
| Stearyl alcohol | 5.0 |
| Liquid paraffin | 17.0 |
| POE (20) cetyl ether | 4.0 |
| Glyceryl monostearate | 2.0 |
| Methyl parahydroxybenzoate | 0.1 |
| Butyl parahydroxybenzoate | 0.1 |
| Propylene glycol | 5.0 |
| Purified water | Balance |

### Formulation example 2

To prepare a preparation of the composition shown in Table 2, α-arbutin was added to purified water and dissolved at 70°C. The solution obtained was maintained at this temperature.

On the other hand, the other ingredients shown in Table 2 were mixed and maintained at 70°C. The above-mentioned mixture of α-arbutin and purified water maintained at 70°C was added to the mixture of the other ingredients maintained at 70°C, and the mixture obtained was cooled with stirring to prepare a cream.

**Table 2**

| | % by weight |
|---|---|
| α-Arbutin | 1.0 |
| Polyethylene glycol isostearate | 7.5 |
| Cetanol | 1.0 |
| Liquid paraffin | 7.5 |
| Isopropyl myristate | 7.5 |
| Diethylene glycol monoethyl ether | 10.5 |
| Parahydroxybenzoate ester | 0.5 |
| Propylene glycol | 5.0 |
| Purified water | Balance |

### Formulation example 3

To prepare a preparation of the composition shown in Table 3, α-arbutin was added to purified water and dissolved at 70°C. The solution obtained was maintained at this temperature.

On the other hand, the other ingredients other than locust bean gum shown in Table 3 were mixed and maintained at 70°C.

The above-mentioned mixture of α-arbutin and purified water maintained at 70°C was added to the mixture of the other ingredients maintained at 70°C. The mixture obtained was sufficiently stirred and cooled down to 50°C, homogenized, and further cooled down to 30°C.

Locust bean gum was added to the mixture cooled down to 30°C, and the mixture obtained was stirred and cooled to prepare a lotion.

**Table 3**

| | % by weight |
|---|---|
| α-Arbutin | 0.05 |
| Polyoxyethylene sorbitan monostearate | 5.0 |
| Sorbitan monostearate | 3.0 |
| Glycerin monostearate | 2.0 |
| Cetanol | 2.5 |
| Isopropyl myristate | 8.0 |
| Parahydroxybenzoate ester | 0.5 |
| Propylene glycol | 2.0 |
| Locust bean gum | 2.0 |
| Purified water | Balance |

### [Examples of the evaluation of dermatological topical preparations]

### Evaluation example 1

Inhibitory effects of α-arbutin on tyrosinase were evaluated using tyrosinase from human cells.

Inhibitory effects were evaluated according to the method of Funayama et al [Biosci. Biotech. Biochem., 57 (10) 1666-1669 (1993)] using human tyrosinase instead of mushroom-derived tyrosinase. β-Arbutin (manufactured by Nisshin Flour Milling Co.) was used as the control.

Results obtained are shown in Table 4.

**Table 4**

| | IC₅₀ (mM) |
|---|---|
| α-Arbutin | 1.0 mM |
| β-Arbutin | 9.0 mM |
| IC₅₀ (mM) : concentration of the test material at which it inhibits tyrosinase activity by 50% | |

As clearly seen from Table 4, α-arbutin of the present invention showed very strong inhibitory effects on human tyrosinase, giving an IC₅₀ value 9 times smaller than that of β-arbutin.

### Evaluation example 2

A clinical trial was conducted using a cream of the present invention which was prepared in the same way as outlined in Example 2 of preparation except that the α-arbutin content was 0.5% by weight, and a control cream of the same composition except that β-arbutin was used at 1.0% by weight, instead of α-arbutin.

Twelve healthy volunteers (6 men and 6 women, 12 person in total aged between 25 and 55) were divided into two groups. A half thereof (3 men and 3 women, 6 person in total) received the cream of the present invention on the medial side of the right arm, while the remaining half of volunteers received the control cream containing β-arbutin at the same site.

Application was made 3 times a day (every 8 hours) for 7 consecutive days. The application sites were exposed to 1 MED (minimal erythema dose) of UV rays using a UVB light source once a day for a total of 3 times starting the first day of application.

Thirty days after completion of this experiment, subjects were crossed and the same experiment was repeated using another site on the medial side of the right arm in order to ensure the homogeneity of the two groups.

The trial was designed as a double-blind study.

The skin blackening prevention effects were evaluated by comparing, with the naked eye, the skin color on the 14th day after starting UV irradiation to the baseline observed before starting irradiation. The following 5-grade scale was used: very effective, effective, slightly effective, ineffective and aggravated.

Results obtained are shown in Table 5.

**Table 5**

| | Control cream (containing 1.0% by weight of β-arbutin) | Invention cream (containing 0.5% by weight of α-arbutin) |
|---|---|---|
| Very effective | 1 (8.3%) | 2 (16.6%) |
| Effective | 3 (25.0%) | 5 (41.6%) |
| Slightly effective | 5 (41.6%) | 4 (33.3%) |
| Ineffective | 3 (25.0%) | 1 (8.3%) |
| Aggravated | 0 | 0 |

As is clear from Table 5, the cream of the present invention containing 0.5% by weight of α-arbutin was more effective than the control cream containing 1.0% by weight of β-arbutin in preventing the skin blackening.

In addition, the dermatological topical preparation of the present invention caused no adverse reactions, indicating that it can be an excellent skin blackening preventing agent.

### Evaluation example 3

A clinical trial was conducted using the ointment obtained in Formulation example 1 of preparation and a control ointment of the same composition except that α-arbutin was replaced with 2.0% by weight of β-arbutin.

Female volunteers (26 persons aged between 40 and 65) with liver spots were divided into 2 homogeneous groups. The ointment of the present invention was applied to one group and the β-arbutin-containing control ointment to the other, both for 3 months, and the efficacy of the two types of ointment for liver spots were compared.

After a 3-month consecutive application, the liver spot improving effects were evaluated with the naked eye using the following 5-grade scale: markedly improved, improved, slightly improved, ineffective and aggravated.

Results are shown in Table 6.

**Table 6**

| | Control ointment (containing 2.0% by weight of β-arbutin) | Invention ointment (containing 2.0% by weight of α-arbutin) |
|---|---|---|
| Markedly improved | 1 (7.6%) | 2 (15.3%) |
| Improved | 3 (23.0%) | 5 (38.4%) |
| Slightly improved | 6 (46.1%) | 4 (30.7%) |
| Ineffective | 3 (23.0%) | 2 (15.3%) |
| Aggravation | 0' | 0 |

As is clear from Table 6, the cream of the present invention containing 2.0% by weight of α-arbutin was more effective than the control cream containing the same amount of β-arbutin.

In addition, the ointment of the present invention caused no adverse reactions, indicating that it can be an excellent liver spot improving agent.

### Evaluation example 4

The stability of α-arbutin in acidic and alkaline regions was evaluated in comparison with hydroquinone.

The decomposition rate was determined in acidic (pH 5.5), neutral (pH 6.5) and alkaline (pH 7.5) aqueous solutions (0.045M and 37°C with both α-arbutin and hydroquinone).

Results are shown in Table 7.

**Table 7**

| Hydroquinone | | | α-arbutin | |
|---|---|---|---|---|
| pH | Decomposition rate constant | Half-life (hrs) | Decomposition rate constant | Half-life (hrs) |
| 5.5 | 0.064 | 10.8 | 0 | Not decomposed* |
| 6.5 | 0.903 | 0.8 | 0.013 | 53.3 |
| 7.5 | 1.117 | 0.6 | 0.022 | 31.5 |

| | | | | |
|---|---|---|---|---|
| * Determined after 69 hrs | | | | |

As is clear from Table 7, the half-life in alkaline and acidic regions was much longer for α-arbutin than hydroquinone as short as 0.6 to 10.8 hours, indicating that α-arbutin is very stable.

Therefore, it can be concluded that the α-arbutin of the present invention should be very stable in dermatological topical preparations.

### Evaluation example 5

The maintenance of α-arbutin on the mouse skin was evaluated as shown below in comparison with β-arbutin.

The back skin was depilated and peeled off after sacrificing mice by cervical dislocation at 24 weeks of age. Samples of the skin were weighed in an amount of 0.5 g and 2.0 g, added to sodium citrate-phosphate buffer solution containing 0.037M α-arbutin or β-arbutin (2.0 mL, pH 7.0), and incubated at 37°C.

The samples were centrifuged 19 and 40 hours later and the amounts of α-arbutin and β-arbutin in the supernatant were determined by HPLC in order to calculate their residual rates.

Each sample was tested in triplicate (n=3) in all of the 4 groups, and mean residual rates were calculated. HPLC was conducted under the following conditions:
Column: ODS (4.6 x 250 mm)
Solvent: methanol-water (20:80, v/v, adjusted to pH 2.5 using phosphoric acid)
Detection: 280 nm
Flow rate: 0.5 mL/min
Temperature: 40°C

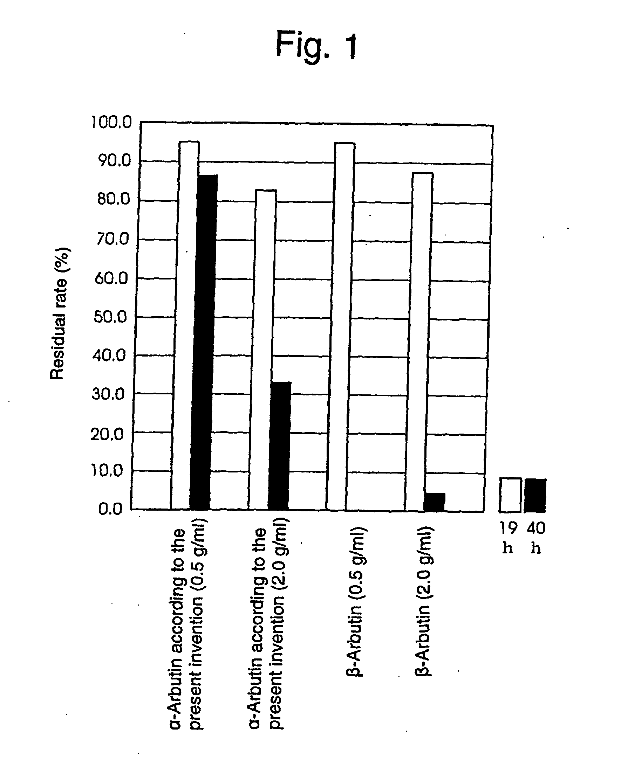

Results obtained are shown in Fig. 1.

As is clear from Fig. 1, both α-arbutin and β-arbutin remained virtually intact 19 hours starting incubation, but decomposition was noted in all groups 40 hours after starting incubation. The residual rate was 87.5% in the group in which α-arbutin was added to 0.5 g of the skin sample. The residual rate was 0% in the group in which β-arbutin was added to the same amount of the skin sample, indicating that β-arbutin was completely decomposed.

In the groups in which 2.0 g of the skin sample was added, α-arbutin showed a residual rate about 10 times larger than that of β-arbutin 40 hours after incubation, indicating that α-arbutin is much less likely to be decomposed than β-arbutin.

### Evaluation example 6

The maintenance of α-arbutin and β-arbutin was evaluated using the same skin samples as in Evaluation example 5.

The skin samples obtained in Evaluation example 5 were weighed, and 2.6 g was added to sodium citrate-phosphate buffer solution (5.2 ml, pH 7.0) and homogenized. The homogenate obtained was used as skin extract fluid.

The skin extract fluid was mixed with citrate-phosphate Na buffer solution containing 0.1M α-arbutin or β-arbutin (5.2 ml, pH 7.0) at a ratio of 1:1 (v/v), and incubated at 37ºC.

The mixtures were centrifuged 4 hours and 20 hours later and the amounts of α-arbutin and β-arbutin in the supernatant were determined by HPLC in order to calculate their residual rates.

Each sample was tested in triplicate (n=3) in both groups, and mean residual rates were calculated.

HPLC was conducted under the conditions used in Evaluation example 5.

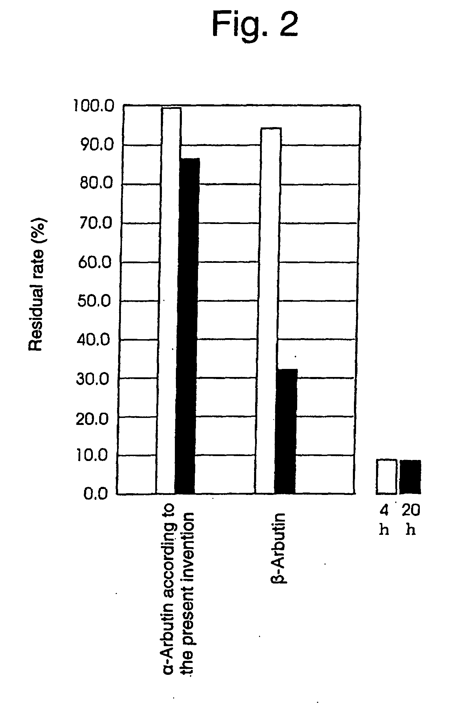

Results obtained are shown in Fig. 2.

As is clear from Fig. 2, both α-arbutin and β-arbutin remained virtually intact 4 hours starting incubation. The residual rate 20 hours after starting incubation was more than twice higher in the α-arbutin group than in the β-arbutin group, indicating α-arbutin is more stable than β-arbutin.

### Utilizability in industry

Thus, the following effects can be obtained with dermatological topical preparations of the present invention containing the α-arbutin of the present invention:
(1) The α-arbutin according to the present invention showed much greater inhibitory effects on tyrosinase, which is closely related to the formation of melanin, which blackens the skin and induces pigmentation, than conventional β-arbutin when these effects were evaluated using human tyrosinase, indicating that it is useful as a whitening agent for use for the human skin.
(2) In clinical trials in healthy volunteers, dermatological topical preparations containing the α-arbutin according to the present invention showed greater whitening effects (prevention of skin blackening) and liver spot improving effects than dermatological topical preparations containing the conventional β-arbutin. No adverse reactions were observed, indicating that there is no problem related to the safety. Therefore, dermatological topical preparations containing the α-arbutin of the present invention are effective as skin whitening agents and liver spot improving agents.
(3) Since the α-arbutin according to the present invention is stable in water, it is effective for use in topical preparations such as ointment, lotion and cream.
(4) Since the α-arbutin according to the present invention is maintained over a ling period of time on the mouse skin, it is effective for use in dermatological topical preparations for humans.

## Claims

1. Dermatological topical preparations containing hydroquinone-α-D-glucopyranoside with superior inhibitory activity against human tyrosinase.
